(19)

**Europäisches Patentamt**

**European Patent Office**

**Office européen des brevets**

(11) **EP 4 613 265 A1**

(12) **EUROPEAN PATENT APPLICATION**

(43) Date of publication:
**10.09.2025 Bulletin 2025/37**

(21) Application number: **24162428.7**

(22) Date of filing: **08.03.2024**

(51) International Patent Classification (IPC):
**A61K 9/127** *(2025.01)* **A61K 47/69** *(2017.01)*
**C12N 9/04** *(2006.01)*

(52) Cooperative Patent Classification (CPC):
**A61K 9/127; C12N 9/0006;** A61K 9/1273

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Alcolase ApS
2200 Copenhagen N (DK)**

(72) Inventors:
- **Mikkelsen, Jeppe Malthe
  2200 Copenhagen N (DK)**
- **Mikkelsen, Mads Emil
  2200 Copenhagen N (DK)**
- **Precht, Mikkel Holstener
  2200 Copenhagen N (DK)**

(74) Representative: **Larsen & Birkeholm A/S
Banegårdspladsen 1
1570 Copenhagen V (DK)**

(54) **LIPOSOMES FOR ENZYMATIC DEGRADATION OF INGESTED ETHANOL**

(57)     The present invention relates to a composition comprising a liposome and/or a polymersome, the liposome and/or the polymersome enclose an enzyme capable of degrading ethanol, wherein the liposome and/or the polymersome fulfils at least one of the following criteria (i) wherein the concentration of enzyme is above 100 U/ml composition; (ii) wherein the liposome and/or the polymersome comprises a particle size of at least 100 nm; (iii) wherein the composition is capable of degrading in the range of 50-5000 mM ethanol per hour; (iv) wherein the liposome and/or the polymersome are stable at acidic pH, such as at a pH 5.5 or below; and/or (v) wherein the pH inside the liposome and/or the polymersome is above pH 6.0.

**Figure 1**

Liposome encapsulated enzymes (ADH) are protected in pH 4

- Liposome w. enzym and detergent pH 4
- Liposome w. enzym w.o detergent pH 4
- Enzyme pH 4

EP 4 613 265 A1

## Description

### Technical field of the invention

The present invention relates to the fields of enzyme technology and to liposomal systems and products for degradation of ethanol. In particular the present invention relates to liposomes and polymersomes comprising enzymes for the degradation of ethanol in the stomach of humans.

### Background of the invention

The global alcoholic beverage market is expected to continuously growing considerable in the future. The demand is strongly driven by the significant rise in demand for alcoholic beverages which originates from the millennial use and culture across the world.

Ethanol is the alcohol compound that is found in most alcoholic drinks. After intake the small parts of the ethanol may be absorbed into the blood stream when going through the body.

When entering the bloodstream, ethanol has various effects on the human body. These effects are mainly described as negative in the literature and may include depressive effects on the brain, uncontrolled behaviour, and increased risk of cardiovascular diseases and cancer are some of them.

Despite this negative effects, alcohol/ethanol is enjoyed by many people and is consumed all around the world. It is difficult to give hard estimates about ethanol absorption and metabolism as it varies depending on genetics, diet, condition, and many other factors. Normally the maximum blood alcohol concentration is reached after 30 minutes from intake.

Ethanol absorption occurs through the whole gastrointestinal tract, but ethanol is mainly absorbed in the small intestine, in particular in the particularly in the duodenum and jejunum, due to the large absorption area (villi structure). The ethanol is mainly degraded in the liver where the rate of degradation is dependent on e.g. the amount of ethanol intake.

Ethanol is toxic and is responsible for about 2.8% of all deaths worldwide. Ethanol is even more dangerous for people with ALDH2 deficiency, i.e. Alcohol Intolerance. Alcohol Intolerance is a genetic condition most common in East-Asia (30-50% of the population). The condition is caused by a genetic mutation which impairs the metabolism of acetaldehyde, which is the byproduct from ethanol degradation. This results in several symptoms such as facial flushing, rapid heartbeat, nausea and an increased risk of cancer.

Many people are therefore having difficulties consuming alcoholic drinks, either because they get alcohol flush or they would simply like to circumvent the effects of alcohol when drinking.

One option is to resort to non-alcoholic drinks, but these may not be available at the place and time of the consumption of alcoholic drinks. Further, the non-alcoholic drinks may taste different than their alcoholic equivalents.

Another option that has been proposed is the use of enzymes for the degradation of ethanol and optionally degradation of acetaldehyde. Currently there are a lot of products trying to solve this problem. The market is however quite fragmented. A few of these products are based on enzyme solutions and none of these enzyme-based solutions has yet penetrated the market.

Others have touched upon liposomal encapsulation of alcohol dehydrogenase (ADH). US 2021/0213107 describes compositions of microparticles comprising e.g. ADH to degrade ethanol. The compositions are for delivery to the blood, so as to degrade ethanol in the blood. Additionally, Yoshimoto et al. (2008) disclose the encapsulation of ADH in liposomes.

There remains a need for more simple and self-administrable products which render it potentially safer and healthier to enjoy alcoholic drinks.

### Summary of the invention

The object of the present invention is to provide compositions which render it safer and healthier to enjoy alcoholic drinks, both for people tolerating alcohol as well as people having alcohol intolerance. A further object of the invention is to provide a composition which may be administered by an individual without the need of medical personnel. It is a further object of the invention that the compositions upon ingestion can withstand the harsh acidity and proteolytic environment in the stomach. It is an even further object of the invention that the compositions upon ingestion can perform and execute the activity in the stomach before the ethanol reaches the small intestine and is absorbed into the blood stream.

Thus, one aspect of the invention relates to a composition comprising a liposome and/or a polymersome, the liposome and/or the polymersome enclose an enzyme capable of degrading ethanol, wherein the liposome and/or the polymersome fulfils at least one of the following criteria:

    i. wherein the concentration of enzyme is above 100 U/ml composition;

ii. wherein the liposome and/or the polymersome comprises a particle size of at least 100 nm;

iii. wherein the composition is capable of degrading in the range of 50-5000 mM ethanol per hour;

iv. wherein the liposome and/or the polymersome are stable at acidic pH, such as at a pH 5.5 or below; and/or

v. wherein the pH inside the liposome and/or the polymersome is above pH 6.0.

Another aspect of the present invention relates to a unit dose comprising a composition according to the present invention, and wherein the unit dose comprising an amount of enzyme, enclosed in a liposome and/or a polymersome, of 50 U/unit dose or above.

Yet another aspect of the present invention relates to a composition, e.g. an ingestible composition, for degrading ethanol in the stomach which comprises liposomes and/or polymersomes that enclose an enzyme degrading ethanol, wherein said liposomes and/or polymersomes are stable at acidic pH.

Still another aspect of the present invention relates to a method for preparing composition, e.g. an ingestible composition, for degrading ethanol in the stomach which comprises liposomes and/or polymersomes that enclose an enzyme degrading ethanol, said method comprising the steps of:

a) dissolving a phospholipid in an organic solvent,

b) hydrating the phospholipids with an aqueous solution of at least one enzyme degrading ethanol,

c) freezing said hydrated lipids in liquid nitrogen,

d) performing at least 3 cycles of thawing in aqueous solution at 20 °C and freezing in liquid nitrogen,

e) optionally performing an extrusion to obtain a more uniform size, to obtain said liposomes and/or polymersomes.

A further aspect of the present invention relates to the use of a composition, e.g. an ingestible composition, according to the present invention, for the degradation of ingested ethanol in the stomach.

An even further aspect of the present invention relates to a kit of parts comprising the composition according to the present invention and instructions for the use according to the present invention.

Yet an aspect of the present invention relates a composition according to the present invention for use in the treatment, prevention or alleviation of alcohol flush.

**Brief description of the figures**

Figure 1 shows the activity of alcohol dehydrogenase (ADH) encapsulated in liposomes. Lower curve: Enzyme pH 4. Middle curve: Liposome with enzyme without detergent pH4. Upper curve: Liposome with enzyme and detergent pH 4.

Figure 2 shows the alcohol oxidase (AOX) activity encapsulated in liposomes. Graph showing the time-dependent production of $H_2O_2$ from oxidation of 50 mM ethanol catalyzed by liposome encapsulated *Pichia pastoris* alcohol oxidase (pAOX) in phosphate buffer, pH 3. (upper line).

Figure 3 shows the ADH and aldehyde dehydrogenase (ALDH) activity outside liposome (free). Time in seconds is shown on the x-axis and absorbance measured at 340 nm on the y-axis. The absorbance corresponds to NADH production. Samples were measured in triplicates each minute for 5 minutes. The different enzyme samples were dissolved in Tris-HCl buffer at pH 8.5 containing NAD+ and ethanol. The linear trendline is shown for each sample. This clearly shows that ALDH can break down acetaldehyde to acetate.

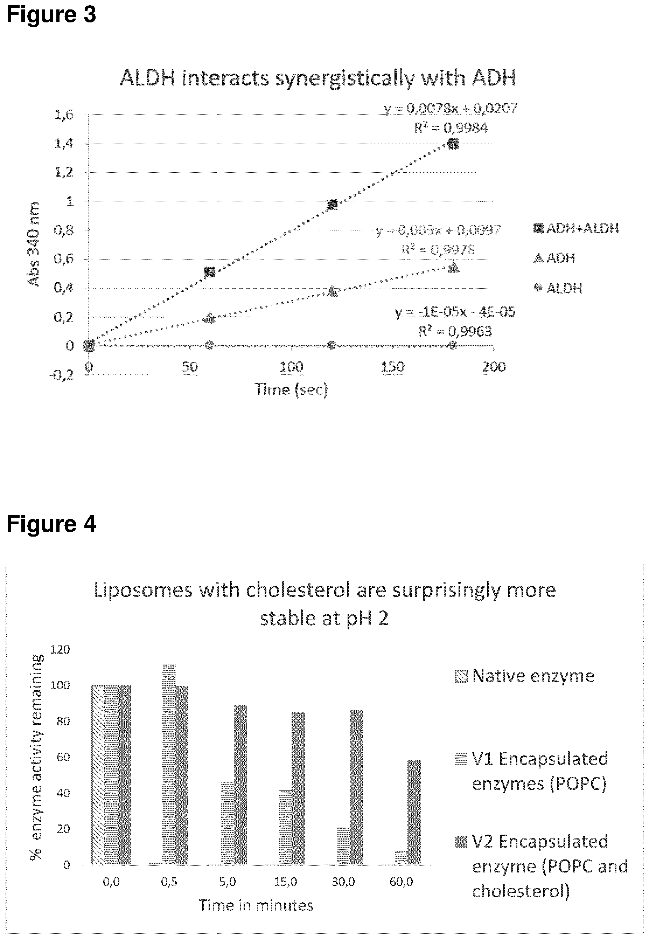

Figure 4 shows the stability of enzymes after being dissolved in an acidic buffer for different amounts of time. The x-axis shows how many minutes the enzymes were treated with pH 2. The y-axis shows the retained activity in % of 0 minutes at pH 2. After 30 minutes at pH 2, V2 retains ~100% activity vs native enzymes 0,2%. V2 liposome consists of 30% cholesterol and 70 % POPC, while V1 consists of only POPC in the membrane. In all cases the enzyme is ADH. The native enzyme (grey) is not encapsulated and close to 0% activity remains after just half a minute in pH 2. V1 and V2 liposomes with ADH shows activity after 60 minutes at in pH 2.

Figure 5 shows the NADH peroxidase activity where both samples contained ALDH, AOX, 1% ethanol, 40 mM phosphate buffer pH 7.5, and limiting amount of NAD$^+$.

Figure 6 shows the results from liposomes with an enzyme system composed of AOX, ALDH, and NADH peroxidase (NP) that were tested at pH 3.

Figure 7 shows the ethanol degradation resulting from an enzyme system composed of AOX, ALDH and NADH peroxidase (NP), in pH 7.5 non-encapsulated.

Figure 8 shows an enzymatic ethanol degradation in a stomach model.

Figure 9 shows ethanol degradation by liposomes loaded with AOX, catalase (CAT), and the combination of AOX and CAT (MIX).

The present invention will now be described in more detail in the following.

**Detailed description of the invention**

The inventors of the present invention surprisingly found a composition that enables people with alcohol intolerance to be able to drink alcohol with less symptoms or even no symptoms.
This was facilitated by the compositions of the present invention which may break down ethanol in the stomach before it is absorbed into the blood and thus distributed throughout the body.
Hence, the composition of the present invention may shift ethanol metabolism from the liver to the stomach.
In this way alcoholic drinks may be enjoyed as if they were close to alcohol-free. This can be relevant for people with alcohol flush but also for people who would like to circumvent the effects of alcohol when drinking.
The composition according to the present invention may also enable that it is potentially safer and healthier to enjoy alcoholic drinks.
It has surprisingly been found that despite a harsh environment with acidity and proteolytic activity as found in the stomach, an effective degradation of ethanol can be achieved by the ingestible compositions of the present invention.
The terms "degrading ethanol", "degradation or ethanol" and the like sas used herein is intended to mean any chemical reaction which converts ethanol into another chemical compound. Non-limiting examples of degrading ethanol is the conversion of ethanol into acetaldehyde, into acetic acid, into carbon dioxide and the like.
Hence, the liposome and/or the polymersome present in the composition according to the present invention may preferably be configured to mainly execute the degradation of ethanol in the stomach of a human.
The practice of the present invention will employ, unless otherwise indicated, conventional techniques of enzymology and liposome technology, which are within the skill of the art. Such techniques are explained fully in the literature. See, for example, the cited references herein.
Accordingly, a preferred embodiment of the present invention relates to a composition comprising a liposome and/or a polymersome, the liposome and/or the polymersome enclose an enzyme capable of degrading ethanol, wherein the liposome and/or the polymersome fulfils at least one of the following criteria:

i. wherein the concentration of enzyme is above 100 U/ml composition;

ii. wherein the liposome and/or the polymersome comprises a particle size of at least 100 nm;

iii. wherein the composition is capable of degrading in the range of 50-5000 mM ethanol per hour, preferably per unit dose composition;

iv. wherein the liposome and/or the polymersome are stable at acidic pH, such as at a pH 5.5 or below; and/or

v. wherein the pH inside the liposome and/or the polymersome is above pH 6.0.

Another preferred embodiment of the present invention relates to a composition comprising a liposome and/or a polymersome, the liposome and/or the polymersome enclose an enzyme capable of degrading ethanol, wherein the concentration of enzyme is above 100 U/ml composition.
Yet another preferred embodiment of the present invention relates to a composition comprising a liposome and/or a polymersome, the liposome and/or the polymersome enclose an enzyme capable of degrading ethanol, wherein the liposome and/or the polymersome comprises a particle size of at least 100 nm.

An even further preferred embodiment of the present invention relates to a composition comprising a liposome and/or a polymersome, the liposome and/or the polymersome enclose an enzyme capable of degrading ethanol, wherein the composition is capable of degrading in the range of 50-5000 mM ethanol per hour, preferably per unit dose composition.

Preferably at least two of the criteria may be fulfilled, even more preferably at least 3 of the criteria are fulfilled, even more preferably at least 4 of the criteria are fulfilled, even more preferably all 5 criteria are fulfilled.

In an embodiment of the present invention criteria (i) may be fulfilled.

In a further embodiment of the present invention criteria (ii) may be fulfilled.

In yet an embodiment of the present invention criteria (i) in combination with criteria (ii) or criteria (iii) may be fulfilled. Preferably, criteria (i) in combination with criteria (ii) and criteria (iii) may be fulfilled.

In an even further embodiment of the present invention criteria (i) in combination with criteria (ii) may be fulfilled.

In another embodiment of the present invention criteria (ii) in combination with criteria (iii) may be fulfilled.

The liposome and/or the polymersome according to the present invention may preferably comprise unilamellar liposome and/or the polymersome.

Liposomes are generally small vesicles that are formed by lipids such as cholesterol and/or phospholipids. They may consist of at least one lipid membrane with a hydrophilic core. They have either a one layer or a bilayer membrane, and can generally vary in size from 20-2000 nanometers, but may be even larger. Liposomes and methods for preparing them are further described in e.g. Akbarzadeh et al. (2013).

The liposome according to the present invention may relate to small vesicles that may be formed by lipids such as cholesterol or phospholipids. The liposome according to the present invention may comprise either a one-layer or a bilayer membrane. They may vary in size but normally measures 20-5000 nanometers (nm) in diameter.

Polymersomes are also vesicles quite alike liposomes. However, despite their similar amphiphilic nature, liposomes and polymersomes exhibit important physical differences. Most notably the molecular weight of building compounds which may be 100-1000 g/mole for liposomes whereas for polymersomes they may be 1000-10000 g/mole. Phospholipids are typically used for liposomes whereas an amphiphilic block copolymer are typically used for polymersomes. Another difference may be the membrane thickness for liposomes is 3-5 nm whereas the membrane thickness for polymersomes is 5-50 nm.

Small molecules such as ethanol, acetaldehyde and acetate can easily diffuse through the cell-membrane of the liposome and/or the polymersome according to the present invention. Additionally, lipid membranes will not allow protons to flow through due to the hydrophobicity of the lipids. Without wishing to be bound by theory, it is believed that these principles are the basis for working of the liposome compositions of the present invention. Conventional liposomes are not necessarily stable in the stomach. They are susceptible to the effect of gastric acid, bile salts and pancreatic lipases combined. This leads to a reduced number of intact liposomes.

The liposome and/or the polymersome according to the present invention may be spherically shaped and hence particle size of the liposome and/or the polymersome may be determined as the diameter of the spherical shaped liposome and/or the polymersome.

Preferably, the liposome and/or the polymersome according to the present invention comprises a particle size above 100 nm, such as a particle size above 200 nm, e.g. above 300 nm, such as a particle size above 400 nm, e.g. above 500 nm, such as a particle size above 600 nm, e.g. above 700 nm, such as a particle size above 800 nm, e.g. above 900 nm, such as a particle size above 1000 nm, e.g. above 1200 nm, such as a particle size above 1500 nm, e.g. above 2000 nm, such as a particle size above 2500 nm, e.g. above 3000 nm, such as a particle size above 3500 nm, e.g. above 4000 nm, such as a particle size above 4500 nm, e.g. above 5000 nm, such as in the range of 100-5000 nm, e.g. in the range of 200-4500 nm, such as in the range of 300-4000 nm, e.g. in the range of 500-3500 nm, such as in the range of 800-3000 nm, e.g. in the range of 900-2500 nm, such as in the range of 1000-2000 nm, e.g. in the range of 1200-1500 nm.

Preferably, the liposome and/or the polymersome may comprise a concentration of enzyme of 50 U/ml composition or above, e.g. 75 U/ml composition or above, such as 100 U/ml composition or above, e.g. 125 U/ml composition or above, such as 150 U/ml composition or above, e.g. 175 U/ml composition or above, such as 200 U/ml composition or above, e.g. 250 U/ml composition or above, such as 300 U/ml composition or above, e.g. 350 U/ml composition or above, such as 400 U/ml composition or above, such as 500 U/ml composition or above, such as 600 U/ml composition or above, e.g. 700 U/ml composition or above, such as 800 U/ml composition or above, e.g. 900 U/ml composition or above, such as 1500 U/ml composition or above, e.g. 2000 U/ml composition or above, such as 3000 U/ml composition or above, e.g. 4000 U/ml composition or above, such as 5000 U/ml composition or above, such as in the range of 50-5000 U/ml composition, e.g. in the range of 75-4000 U/ml composition, such as in the range of 100-3000 U/ml composition, e.g. in the range of 125-2000 U/ml composition, such as in the range of 150-1500 U/ml composition, e.g. in the range of 175-1000 U/ml composition, such as in the range of 200-800 U/ml composition, e.g. in the range of 250-600 U/ml composition, such as in the range of 300-400 U/ml composition.

Alternatively, the liposome and/or the polymersome may comprise a concentration of enzyme of such as in the range of 50-5000 U/ml composition, e.g. in the range of 125-4000 U/ml composition, such as in the range of 250-3500 U/ml composition, e.g. in the range of 500-3000 U/ml composition, such as in the range of 1000-2500 U/ml composition, e.g. in the range of 1500-2000 U/ml composition.

The term U/ml may relate to the concentration of enzyme (ethanol degrading enzyme or enzyme capable of degrading ethanol, which may be used interchangeably) relative to the amount of the composition.

In the context of the present invention the terms "U" or "Unit" may be used interchangeably and may be calculated based on that one unit may oxidize 1.0 $\mu$mole of ethanol to ethyl-aldehyde per minute at optimum pH for the enzyme in question (preferably at pH 7.5) at 25°C.

In an embodiment of the present invention the liposome and/or the polymersome comprises a particle size above 100 nm and the liposome and/or the polymersome enclose an enzyme capable of degrading ethanol, and wherein the liposome and/or the polymersome is provided with a concentration of enzyme above 100 U/ml composition.

In a further embodiment of the present invention the activity of the enzyme (in particular the specific activity of the enzyme) in the liposome and/or the polymersome may be in the range of 1-500 U/mg protein, such as in the range of 10-450 U/mg protein, e.g. in the range of 25-400 U/mg protein, such as in the range of 50-375 U/mg protein, e.g. in the range of 100-350 U/mg protein, such as in the range of 200-325 U/mg protein, e.g. in the range of 250-310 U/mg protein, e.g. about 300 U/mg protein, when the protein is a an ethanol degrading enzyme, e.g. ADH or AOX.

In a further embodiment of the present invention the activity of the enzyme (in particular the specific activity of the enzyme) in the liposome and/or the polymersome may be in the range of 1-10000 U/mg protein, such as in the range of 100-9000 U/mg protein, e.g. in the range of 250-8000 U/mg protein, such as in the range of 500-7000 U/mg protein, e.g. in the range of 500-6500 U/mg protein, such as in the range of 1000-6000 U/mg protein, e.g. in the range of 2000-5500 U/mg protein, e.g. in the range of 4000-5200 U/mg protein, such as in the range of 2000-5500 U/mg protein, e.g. about 5000 U/mg protein, when the protein may be catalase or the like.

The activity or the specific activity of the enzyme may be determined as the activity of an enzyme per milligram of total protein over time (e.g. expressed as $\mu$mol/min per mg protein).

The improved activity of the enzyme (in particular the specific activity of the enzyme) in the liposome and/or the polymersome and/or the improved concentration of enzyme in the liposome and/or the polymersome, may provide improved digestibility of the ethanol in the stomach.

In an embodiment of the present invention, the composition according to the present invention may be capable of degrading in the range of 50-5000 mM ethanol per hour, such as in the range of 100-4000 mM ethanol per hour, e.g. in the range of 150-3000 mM ethanol per hour, such as in the range of 200-2500 mM ethanol per hour, e.g. in the range of 300-2000 mM ethanol per hour, such as in the range of 400-1800 mM ethanol per hour, e.g. in the range of 500-1500 mM ethanol per hour, such as in the range of 600-1300 mM ethanol per hour, e.g. in the range of 750-1200 mM ethanol per hour, such as in the range of 900-1100 mM ethanol per hour, e.g. in the range of 950-1000 mM ethanol per hour.

Preferably, the degradation of ethanol per hour mentioned above may be determined per unit dose as described herein.

Preferably, the term "degrading" of ethanol according to the present invention may relate to the conversion of ethanol to acetaldehyde, acetic acid, or the like.

The liposomes and/or polymersomes according to the present invention may be active and degrade the ethanol in the stomach of a human rather than in the blood or liver of the human body.

The liposomes and/or polymersomes according to the present invention may be provided with a stability at different pH ranges. Preferably, the liposomes and/or polymersomes may be provided with a stability against an inside pH (e.g. a stability against an inside pH in the range of pH 6.0-8.5), and against an outside pH (e.g. a stability against an outside pH in the range of pH 2.0-5.0).

In an embodiment of the present invention the liposome and/or the polymersome may be stable at pH in the range of pH 2.0-8.5, such as in the range of pH 2.5-8.0, e.g. in the range of pH 3.0-7.5.

Preferably, the pH inside the liposome and/or the polymersome may be above pH 6.0, such as pH 6.5 or above, e.g. pH 7.0 or above, such as pH 7.5 or above, e.g. pH 8.0 or above, such as pH 8.2 or above, e.g. pH 8.5 or above.

The composition according to the present invention may comprise liposomes and/or polymersomes which may be stable at acidic pH.

The term "stable at acidic pH" may relate to the stability of the liposomes and/or polymersomes when being subjected to an outside environment having an acidic pH.

In an embodiment of the present invention the term "stable at acidic pH" may be used in connection with liposomes or polymersomes and may relate to the structure of the liposomes or polymersomes that may remain intact at acidic pH so that the one or more enzymes within said liposomes or polymersomes remain active. Acidic pH is pH values typical for the human stomach such as pH 3.0 or pH 2.0. The pH in the stomach might be higher depending on the intake of food and drinks. Preferably, the term "stable at acidic pH" means that a liposome or polymersome that enclose an enzyme capable of degrading ethanol, and which undergoes the test specified in Example 4, retains at least 20% of the enzyme activity after 15 minutes.

This stability at acidic pH may be provided to allow for the enzymes to be degrade the ethanol in the stomach of a human being without the liposomes and/or polymersomes collapse or degrade by the environment of the stomach fluid of a human stomach comprising a range of acids and proteases.

Preferably, the liposome and/or the polymersome may be stable at an acidic pH, preferably at a pH 5.5 or below, e.g. at pH 5.0 or below, such as pH 4.5 or below, e.g. pH 4.0 or below, such as pH 3.5 or below, e.g. pH 3.0 or below, such as below pH 2.5, e.g. below pH 2.0.

The stability, e.g. the acidic stability, may be provided for a sufficient time period to degrade at least part of the ethanol consumed.

Preferably, the liposome and/or the polymersome may be stable for a period of 20-300 minutes, such as for a period of 25-240 minutes, e.g. for a period of 30-180 minutes, such as for a period of 35-160 minutes, e.g. for a period of 40-140 minutes, such as for a period of 45-120 minutes, e.g. for a period of 50-100 minutes, such as for a period of 55-90 minutes, e.g. for a period of 58-80 minutes, such as for a period of 60-70 minutes.

Preferably, the liposome and/or the polymersome may be stable at an acidic pH (in particular at a pH below pH 5.5 or below, e.g. at pH 5.0 or below, such as pH 4.5 or below, e.g. pH 4.0 or below, such as pH 3.5 or below, e.g. pH 3.0 or below, such as below pH 2.5, e.g. below pH 2.0) for a sufficient time period to degrade at least part of the ethanol consumed (e.g. for a period of 20-300 minutes, such as for a period of 25-240 minutes, e.g. for a period of 30-180 minutes, such as for a period of 35-160 minutes, e.g. for a period of 40-140 minutes, such as for a period of 45-120 minutes, e.g. for a period of 50-100 minutes, such as for a period of 55-90 minutes, e.g. for a period of 58-80 minutes, such as for a period of 60-70 minutes).

In an embodiment of the present invention the liposome and/or polymersome:

- having an inside pH above pH 6.0, such as pH 6.5 or above, e.g. pH 7.0 or above, such as pH 7.5 or above, e.g. pH 8.0 or above, such as pH 8.2 or above, e.g. pH 8.5 or above;
- being stable when subjected to an outside environment having a pH below pH 5.5 or below, e.g. at pH 5.0 or below, such as pH 4.5 or below, e.g. pH 4.0 or below, such as pH 3.5 or below, e.g. pH 3.0 or below, such as below pH 2.5, e.g. below pH 2.0); and
- maintain the stability for a period of 20-300 minutes, such as for a period of 25-240 minutes, e.g. for a period of 30-180 minutes, such as for a period of 35-160 minutes, e.g. for a period of 40-140 minutes, such as for a period of 45-120 minutes, e.g. for a period of 50-100 minutes, such as for a period of 55-90 minutes, e.g. for a period of 58-80 minutes, such as for a period of 60-70 minutes.

The composition according to the present invention may be an ingestible composition.

The term "ingestible composition" as used herein is intended to mean a composition which is suitable for being ingested by humans, i.e. taken in for or as if for digestion by a human. An ingestible composition may be a solid composition, a liquid composition, a dry composition or the like. Non-limiting examples of ingestible compositions may be a food product, a beverage, a tablet, a pill or a liquid liposome and/or polymersome composition.

In another embodiment of the present invention the composition may be a liquid composition, in particular the liquid composition may be an aqueous composition.

The liquid composition according to the present invention may comprise liposomes enclosing enzymes capable of degrading ethanol, which liposomes may be suspended in the liquid composition.

In an embodiment of the present invention the liquid phase surrounding the liposomes and/or polymersomes of a liquid composition according to the present invention may comprise a buffer.

In a further embodiment of the present invention the liquid phase surrounding the liposomes and/or polymersomes of a liquid composition according to the present invention may comprise a preservative.

In an embodiment of the present invention the liquid phase, preferably an aqueous phase, within said liposomes and/or polymersomes may be substantially isotonic with a liquid phase, preferably an the aqueous phase, outside said liposomes and/or polymersomes.

In an embodiment of the present invention the composition the composition may be a dried composition, a powdered composition or a pill/tablet comprising the composition.

Preferably, the dried composition, a powdered composition or a pill/tablet comprising the composition may be provided by applying various drying techniques such as freeze drying, spray drying, spray-freeze drying, electrohydrodynamic, and supercritical methods.

When the composition according to the present invention may be provided as a dried composition and/or as a powdered composition, the dried composition and/or as the powdered composition may be resuspended in a liquid component, in particular an aqueous component, before consumption.

Liposomes may be formed from a wide variety of lipids, such as phosphatidylcholines. Useful phosphatidylcholines (PC) may be dimyristoyl phosphatidylcholine (DMPC), dipalmitoylphosphatidylcholine (DPPC), distearoylphosphatidylcholine (DSPC), dioleoyl phosphatidylcholine (DOPC) 1-palmitoyl-2-oleoyl-sn-glycero-3-phosphocholin (POPC), or naturally extracted phosphatidylcholine (PC). Phosphatidylcholine may be obtained from a range of different sources, including plant materials or animal materials. The plant material may preferably be selected from soy, or sunflower. The animal material may be a material obtained from cattle (e.g. beef or milk), from fish, or from chicken (in particular egg). Preferably, the animal material may be eggs.

Preferably, the liposome comprises phosphatidylcholine obtained from soy, sunflower and/or eggs.

Useful phosphatidylglycerols (PG) are dimyristoyl phosphatidylglycerol (DMPG), dipalmitoylphosphatidylglycerol (DPPG), distearoyl phosphatidylglycerol. (DSPG) and dioctanoyl phosphatidylglycerol (DOPG). Useful phosphatidyl ethanolamines (PE) may be 1,2-dimyristoyl-sn-glycero-3-phosphoethanolamine (DMPE) and 1,2-dipalmitoyl-sn-glycero-3-phosphoethanolamine (DPPE). Useful phosphatidylserines (PSe) may be dimyristoyl phosphatidylserine (DMPS) and dipalmitoyl phosphatidylserine (DPPS). A useful sphingomyelin (SM) may be palmitoyl sphingomyelin.

Further useful lipid components of liposome and/or polymersome which may help modulate the properties may be sterols. The modulated properties may be enhancing the stability of the liposomes.

Sterols are amphipathic lipids, occurring in most eukaryotes including animals, plants and fungi. Sterols have a gonane structure with the formula $C_{17}H_{28}O$, being derived from gonane by replacement of a hydrogen atom in position 3 by a hydroxyl group.

Preferably, the at least one sterol may be selected from the group consisting of cholesterol, phytosterol, lanosterol, and ergosterol. Preferably, the at least one sterol may be cholesterol or phytosterol. Even more preferably the at least one sterol may be cholesterol.

In an embodiment said liposome or polymersome contains said at least one sterol is in an amount in the range from 10% to 60% (mol/mol) of said liposome or polymersome, in the range of 10% to 50% (mol/mol) of said liposome or polymersome, in the range from 15% to 40% (mol/mol) of said liposome or polymersome or range from 20% to 35% (mol/mol) of said liposome or polymersome, e.g. about 30% (mol/mol) of said liposome or polymersome.

The liposome and/or polymersome according to the present invention may comprises in the range of 40-100% (mol/mol) phospholipids, such as in the range of 50-90% (mol/mol) phospholipids, e.g. in the range of 60-80 % (mol/mol) phospholipids, such as about 70% (mol/mol) phospholipids.

In an embodiment of the present invention the liposome and/or polymersome comprise in the range of 10-60% (mol/mol) sterol (preferably cholesterol), such as in the range of 15-50% (mol/mol) sterol (preferably cholesterol), e.g. in the range of 20-40% (mol/mol) sterol (preferably cholesterol), such as in the range of 25-35% (mol/mol) sterol (preferably cholesterol), e.g. about 30% (mol/mol) sterol (preferably cholesterol), and in the range of 40-100% (mol/mol) phospholipids, such as in the range of 50-90% (mol/mol) phospholipids, e.g. in the range of 60-80% (mol/mol) phospholipids, such as about 70% (mol/mol) phospholipids.

There are a number of enzymes known for degrading ethanol and they originate both from prokaryotic and eukaryotic cells. Alcohol dehydrogenase (ADH) catalyses the oxidation of alcohols into aldehyde or ketones, e.g. by catalysing the reaction:

$$Ethanol + NAD^+ \rightarrow Acetaldehyde + NADH + H^+$$

The electron acceptor is nicotinamide adenine dinucleotide ($NAD^+$). It is to be understood that liposomes or polymersomes that enclose an ADH for degrading ethanol at the time of degrading ethanol must also comprise $NAD^+$ or another suitable electron acceptor.

In an embodiment of the present invention the composition comprises liposomes or polymersomes that enclose ADH and $NAD^+$.

In another embodiment the composition comprises liposomes or polymersomes that enclose ADH and an enzyme which can regenerate $NAD^+$ from NADH.

In another embodiment the composition comprises liposomes or polymersomes that enclose ADH, $NAD^+$ and an enzyme which can regenerate $NAD^+$ from NADH.

Regeneration of $NAD^+$ from NADH may be catalyzed by a number of enzymes, e.g. NADH oxidases which may catalyse the reactions:

$$NADH + H^+ + O_2 \rightarrow NAD^+ + H_2O_2$$

$$NADH + H^+ + \tfrac{1}{2}O_2 \rightarrow NAD^+ + H_2O$$

In another embodiment the composition according to the present invention may comprise liposomes and/or polymersomes that enclose ADH and NADH oxidase.

In another embodiment the composition comprises liposomes and/or polymersomes that enclose ADH, $NAD^+$ and NADH oxidase.

Another option to regenerate NAD+ is NADH peroxidase which catalyses the reaction:

$$NADH + H_2O_2 \rightarrow NAD^+ + 2\ H_2O$$

NADH peroxidase may be found in *Streptococcus faecalis* ATCC 11700 and is commercially available from e.g. NZYTech (catalogue number AE00201). In the present invention NADH peroxidase may be particularly useful when used in combination with an oxidase that makes the hydrogen peroxide, e.g. AOX or ALOX.

It is to be understood that dehydrogenases may be used which employ a different redox system than $NAD^+$/NADH. One such system may be the ubiquinone/Ubiquinol system.

Alcohol dehydrogenase (quinone) may be an enzyme with systematic name alcohol:quinone oxidoreductase which catalyses the reaction:

$$Ethanol + Ubiquinone \rightarrow Acetaldehyde + Ubiquinol$$

Since the acetaldehyde from the degradation of ethanol by ADH in the digestive system may cause various undesirable effects, it is desirable also to degrade acetaldehyde. One way may be to oxidize acetaldehyde into acetic acid which is abundant in foods and do not have the same undesirable effects.

The degradation of acetaldehyde can be done using aldehyde dehydrogenase (ALDH) which catalyses the reaction:

$$Acetaldehyde + NAD^+ \rightarrow Acetic\ acid + NADH + H^+$$

In another embodiment the composition comprises liposomes and/or polymersomes that enclose ADH, ALDH and an enzyme which can regenerate $NAD^+$ from NADH.

In another embodiment the composition comprises liposomes and/or polymersomes that enclose ADH, ALDH, $NAD^+$ and an enzyme which can regenerate $NAD^+$ from NADH.

In another embodiment the ingestible composition comprises liposomes or polymersomes that enclose ADH, ALDH and NADH oxidase.

In another embodiment the composition comprises liposomes and/or polymersomes that enclose ADH, ALDH, $NAD^+$ and NADH oxidase.

An alternative option for degradation of acetaldehyde is aldehyde oxidase (ALOX) which catalyses the reaction:

$$Aldehyde + H_2O + O_2 \rightarrow Carboxylate + H_2O_2 + H^+$$

Here the hydrogen peroxide can be eliminated by natural reactions in the stomach and intestines, but it can also rapidly be eliminated by catalase (CAT) which may be a highly effective enzyme. Catalase (CAT) may catalyse the reaction:

$$2\ H_2O_2 \rightarrow 2\ H_2O + O_2$$

In an embodiment the composition comprises liposomes and/or polymersomes that enclose ADH, ALOX and an enzyme which can regenerate $NAD^+$ from NADH.

In another embodiment the composition comprises liposomes and/or polymersomes that enclose ADH, ALOX, CAT and an enzyme which can regenerate $NAD^+$ from NADH.

In another embodiment the composition comprises liposomes and/or polymersomes that enclose ADH, ALOX, $NAD^+$ and an enzyme which can regenerate $NAD^+$ from NADH.

In another embodiment the composition comprises liposomes and/or polymersomes that enclose ADH, ALOX, CAT, $NAD^+$ and an enzyme which can regenerate $NAD^+$ from NADH.

In another embodiment the composition comprises liposomes and/or polymersomes that enclose ADH, ALOX and NADH oxidase.

In another embodiment the composition comprises liposomes and/or polymersomes that enclose ADH, ALOX, CAT and NADH oxidase.

In another embodiment the composition comprises liposomes and/or polymersomes that enclose ADH, ALOX, $NAD^+$ and NADH oxidase.

In another embodiment the composition comprises liposomes and/or polymersomes that enclose ADH, ALOX, CAT, $NAD^+$ and NADH oxidase.

In yet another embodiment the composition comprises liposomes and/or polymersomes that enclose formate oxidase, e.g. obtained from *Aspergillus oryzae* to degrade acetaldehyde.

In yet another embodiment the composition comprises liposomes and/or polymersomes that enclose ADH and formate oxidase, e.g. from *Aspergillus oryzae,* to degrade acetaldehyde.

In another embodiment the composition comprises liposomes and/or polymersomes that enclose lactate oxidase, e.g. from *Aerococcus viridans,* to degrade acetaldehyde.

In another embodiment the composition comprises liposomes and/or polymersomes that enclose ADH and lactate oxidase, e.g. from *Aerococcus viridans,* to degrade acetaldehyde.

Preferably, the composition comprising liposomes and/or polymersomes according to the present invention may also degrade ethanol by enclosing alcohol oxidase (AOX) which catalyses the reaction:

$$\text{Ethanol} + O_2 \rightarrow \text{Acetaldehyde} + H_2O_2$$

In this embodiment, there is no need for the $NAD^+$/NADH redox system, but rather $O_2$/$H_2O_2$ functions as the redox system. Hence, AOX employing the $O_2$/$H_2O_2$ redox system may preferably be used as an alternative to ADH.

In a further embodiment the composition comprises liposomes and/or polymersomes that enclose alcohol oxidase (AOX).

In another embodiment the composition comprises liposomes and/or polymersomes that enclose AOX and ALOX.

In another embodiment the composition comprises liposomes and/or polymersomes that enclose AOX, ALOX and an enzyme which can eliminate hydrogen peroxide.

In another embodiment the composition comprises liposomes and/or polymersomes that enclose AOX, ALOX and CAT.

In another embodiment the composition comprises liposomes and/or polymersomes that enclose AOX, ALOX and NADH peroxidase.

In another embodiment the composition comprises liposomes and/or polymersomes that enclose AOX and ALDH.

In another embodiment the composition comprises liposomes and/or polymersomes that enclose AOX, ALDH and $NAD^+$.

In another embodiment the composition comprises liposomes and/or polymersomes that enclose AOX, ALDH, $NAD^+$ and an enzyme which can regenerate $NAD^+$ from NADH.

In another embodiment the composition comprises liposomes and/or polymersomes that enclose AOX, ALDH, $NAD^+$ and NADH oxidase.

In another embodiment the composition comprises liposomes and/or polymersomes that enclose AOX, e.g. obtained from *P. chrysosporium.*

In another embodiment the composition comprises liposomes and/or polymersomes that enclose the enzyme AOX, e.g. obtained from *P. chrysosporium* F101S.

In yet another embodiment the composition comprises liposomes and/or polymersomes that enclose AOX, e.g. obtained from *Pichia pastoris* or *Hanseluna spp.*

Preferably, the enzyme capable of degrading ethanol may be alcohol oxidase (AOX).

In a preferred embodiment of the present invention the composition may be an oral preparation comprising the composition according to the present invention, wherein the composition comprising a liposome and/or a polymersome, and wherein the liposome and/or the polymersome enclose an enzyme capable of degrading ethanol.

The composition according to the present invention and/or the oral preparation according to the present invention may preferably be provided for degrading ethanol in the stomach.

The liposomes and/or polymersomes according to the present invention may be partly or fully degraded in the stomach of the human.

In an embodiment of the present invention the partly degraded liposomes and/or polymersomes according to the present invention may relate to a degradation of at least 50% of the liposomes and/or polymersomes in the stomach of the human, such as at least 60% of the liposomes and/or polymersomes, e.g. at least 70% of the liposomes and/or polymersomes, such as at least 80% of the liposomes and/or polymersomes, e.g. at least 90% of the liposomes and/or polymersomes, such as at least 95% of the liposomes and/or polymersomes, e.g. at least 98% of the liposomes and/or polymersomes.

Without being bound by theory, the inventors of the present invention trust that the configuration of the liposome and/or polymersome according to the present invention, ensures that liposomes and/or polymersomes not degraded in the stomach may mainly be secreted via the gastrointestinal tract.

In an embodiment of the present invention the liposome and/or polymersome may preferably not be found in the blood of a person consuming the composition or the oral preparation of the present invention.

Preferably, less than 25% of the content of the liposome and/or polymersome consumed by a human may be found in the blood and/or in the liver of the human consuming the composition according to the present invention, such as less than 20%, e.g. less than 15%, such as less than 10%, e.g. less than 5%, such as less than 2%, e.g. less than 1%, such as less than 0.5%, e.g. less than 0.1%, such as less than 0.01%, e.g. less than 0.001%.

A preferred embodiment of the present invention relates to a unit dose comprising a composition according to the present invention, wherein the unit dose comprising an amount of enzyme, enclosed in a liposome and/or a polymersome, of 50 U/unit dose or above.

The unit dose according to the present invention may be provided in liquid composition (e.g. in an aqueous composition) or in dry composition (e.g. as a tablet or a pill).

In an embodiment of the present invention, the composition may be provided as a liquid composition with a unit dose comprising a volume in the range of 1-200 ml, such as in the range of 5-150 ml, e.g. in the range of 10-100 ml, such as in the range of 20-90 ml, e.g. in the range of 30-80 ml such as in the range of 35-70 ml, e.g. in the range of 40-60 ml, such as in the range of 45-55 ml, e.g. about 50 ml.

In a further embodiment of the present invention, the composition may be provided in a dry composition, e.g. as a pill or a tablet, provided with a unit dose comprising in the range of 50-20000U per pill/tablet liposome and/or polymersome, such as in the range of 100-19000 U per pill/tablet, e.g. in the range of 500-18000 U per pill/tablet, such as in the range of 1000-17000 U per pill/tablet, e.g. in the range of 2000-16000 U per pill/tablet, such as in the range of 3000-15000 U per pill/tablet, e.g. in the range of 4000-14000 U per pill/tablet, such as in the range of 5000-13000 U per pill/tablet, e.g. in the range of 6000-12000 U per pill/tablet, such as in the range of 7000-11000 U per pill/tablet, e.g. in the range of 8000-10000 U per pill/tablet, such as about 9000 U per pill/tablet.

Preferably, the unit dose according to the present invention may comprise an enzyme capable of degrading ethanol which enzyme may be enclosed in the liposome and/or polymersome, and wherein the enzyme may be provided in an amount of 50 U/unit dose or above, e.g. 75 U/unit dose or above, such as 100 U/unit dose or above, e.g. 125 U/unit dose or above, such as 150 U/unit dose or above, e.g. 175 U/unit dose or above, such as 200 U/unit dose or above, e.g. 250 U/unit dose or above, such as 300 U/unit dose or above, e.g. 350 U/unit dose or above, such as 400 U/unit dose or above, e.g. 500 U/unit dose or above, such as 600 U/unit dose or above, e.g. 700 U/unit dose or above, such as 800 U/unit dose or above, e.g. 900 U/unit dose or above, such as 1500 U/unit dose or above, e.g. 2000 U/unit dose or above, such as 3000 U/unit dose or above, e.g. 4000 U/unit dose or above, such as 5000 U/unit dose or above, e.g. 6000 U/unit dose or above, such as 7000 U/unit dose or above, e.g. 8000 U/unit dose or above, such as 9000 U/unit dose or above, e.g. 10000 U/unit dose or above, such as 11000 U/unit dose or above, such as in the range of 50-17500 U/unit dose, e.g. in the range of 100-15000 U/unit dose, such as in the range of 150-12000 U/unit dose, e.g. in the range of 250-10000 U/unit dose, such as in the range of 500-8500 U/unit dose, e.g. in the range of 1000-7500 U/unit dose, such as in the range of 1500-6000 U/unit dose, e.g. in the range of 2000-5500 U/unit dose, such as in the range of 2500-5000 U/unit dose, e.g. in the range of 3000-4500 U/unit dose, such as in the range of 3500-4000 U/unit dose.

Various factors may influence the stability of liposomes and/or polymersomes, such as temperature, pH, surface charge, and/or the lipid composition used for preparing the liposome and/or polymersome.

The stomach is a J-shaped organ, which results in a side with a lesser curvature, and a side with a greater curvature. It is attached to the esophagus, where foods and liquids enter the stomach, and to the duodenum where the processed content leaves the stomach. The inner surface of the stomach consists of irregular folds (rugae) that increases the surface area and allows the stomach to expand upon meals. Around 1.5 L of fluid is secreted from the gastric glands to the stomach every day. Hydrochloric acid (HCl) is secreted to lower the pH to around 2. This ensures that most microorganisms are killed when they enter the stomach. Additional pepsinogen, which is the precursor for pepsin, is secreted into the stomach. Pepsin breaks down proteins in the stomach. Both the extreme acidity and the pepsin inside the stomach represents a harsh environment to enzymes, including most ingestible composition entering the stomach.

To improve stability of the liposome and/or polymersome, various approaches may be implemented to stabilize them. Such methods of liposome and/or polymersome stabilization may include surface modification, stabilizing membrane, changing lipid composition, and the addition of surfactants and nanoparticles.

Some of these methods for liposome and/or polymersome stabilization may include:

- PEGylation (Polyethylene Glycol) or the like, which may involve attaching polyethylene glycol chains to the surface of liposomes and/or polymersomes. PEGylation or the like may increases liposome stability by providing steric hindrance, reducing aggregation and/or opsonization;

- Coating using polysaccharides like hyaluronic acid, dextran, chitosan, and alginate may be used for coating the liposomes and/or polymersomes. These polysaccharide coatings can improve stability, biocompatibility, and targeting capabilities of liposomes. Additionally, polysaccharides can provide sites for ligand conjugation, enabling additional functionalities to the liposome and/or polymersome if found necessary;

- Conjugation or absorption of proteins and/or peptides: Proteins or peptides onto the surface of liposomes and/or polymersomes which may improve stability;

- Lipid bilayer modifications may be provided by incorporating components, such as phospholipids e.g. modified with polyethylene glycol (PEG-lipids), or charged lipids (e.g., positively charged lipids like stearylamine or fusogenic lipids e.g., cholesterol hemisuccinate) into the liposome and/or polymersome bilayers to provide stability and stealth properties.

- Other types of surface-active molecules (like amphiphilic molecules, such as surfactants or block copolymers), or zwitterionic lipids, which may comprise both positively and negatively charged groups, can be adsorbed onto the liposome and/or polymersome and used as coating and may further stabilize the liposome and/or the polymersome.

In an embodiment of the present invention the liposome and/or the polymersome may be provided with one or more surface modifications. Preferably, the one or more surface modifications may be PEGylation, coating (e.g. using polysaccharides), conjugation or absorption of proteins and/or peptides, lipid bilayer modifications, or a combination hereof.

Furthermore, to stabilize the therapeutic compound (including enzymes) nanocarriers can be used. Nanocarriers can have different sizes, functional properties, and ways of stabilization. Nanocarriers is however just one way of stabilizing enzymes. Enzymes can also be stabilized by protein engineering, chemical modification, crosslinking or by adding additives.

By selecting appropriate methods for improving or controlling liposome and/or polymersome stabilization, the inventors of the present invention may tailor for liposome and/or polymersome to stabilisation time period in the stomach and improve the activity in degrading the ethanol present in the stomach of human.

The stability of liposomes and/or polymersome intended to be ingested and entering the stomach has surprisingly been further improved. Liposomes and/or polymersome may for instance be coated with a layer of polymers in order to increase their stability. Another approach may be PEGylation of liposomes and/or polymersome which have been shown to increase circulation time in the blood and in the stomach, it may potentially hinder the pancreatic lipase.

The composition comprising liposomes and/or polymersomes according to the present invention may be prepared in different ways, however, the inventors of the present invention found a preferred method for preparing the composition according to the present invention.

A preferred embodiment of the present invention relates to a method for preparing a composition comprising a liposome and/or a polymersome according to the present invention, said method comprising the steps of:

a) dissolving a phospholipid in an organic solvent,

b) hydrating the phospholipids with an aqueous solution of at least one enzyme degrading ethanol,

c) freezing said hydrated lipids in liquid nitrogen,

d) performing at least 3 cycles of thawing in aqueous solution at 20 °C and freezing in liquid nitrogen,

e) optionally performing an extrusion to obtain a more uniform size,

to obtain a composition comprising the liposomes and/or the polymersomes.

Liposomes may be prepared with the following procedure: 1-palmitoyl-2-oleoyl-sn-glycero-3-phosphocholin (POPC) is dissolved in chloroform and the chloroform is evaporated under a weak nitrogen gas stream followed by vacuum. After complete evaporation of the chloroform the lipids are hydrated to form liposomes. This is done by slowly adding buffer containing the relevant enzymes and optionally cofactors, and freezing in liquid nitrogen and afterwards thawing in water at room temperature (20 °C). The freeze/thaw cycle is repeated seven times to increase unilamellar liposome formation. To obtain more uniform liposomes, an extrusion may be done at e.g. 800 nm. This size secures several enzymes and optional cofactors inside each liposome. The liposome size can be determined with dynamic light scattering (DLS).

A preferred embodiment of the present invention relates to a kit of parts comprising the composition according to the present invention and instructions for the use according to the present invention.

A further preferred embodiment of the present invention relates to the use of a composition according to the present invention, for the degradation of ingested ethanol in the stomach.

The composition according to the present invention may be administered by an individual having alcohol intolerance.

In embodiment of the present invention the composition according to the present invention, for the degradation of ingested ethanol in the stomach wherein an individual having alcohol intolerance self-administers the composition.

The composition according to the present invention may be ingested by swallowing by an individual prior to and/or after the intake or consumption of ethanol, such as an alcoholic beverage.

Preferably, the composition according to the present invention may be taken (preferably by swallowing) in a period from 30 minutes before and/or no later than 30 minutes after the consumption of ethanol, such as one or more alcoholic beverages. Even more preferably, the composition according to the present invention may be taken (preferably by swallowing) in a period from 15 minutes before and/or no later than 15 minutes after the consumption of ethanol, such as one or more alcoholic beverages. Even more preferably, the composition according to the present invention may be taken (preferably by swallowing) in a period from 10 minutes before and/or no later than 10 minutes after the consumption of ethanol, such as one or more alcoholic beverages. Even more preferably, the composition according to the present invention may be taken (preferably by swallowing) in a period from 5 minutes before and/or no later than 5 minutes after the consumption of ethanol, such as one or more alcoholic beverages.

The composition according to the present invention for use in the treatment, prevention or alleviation of alcohol flush.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

## Examples

Example 1 - Ethanol degradation by liposome composition

Ethanol degradation using ADH occurs with $NAD^+$ conversion meaning that the amount of $NAD^+$ determines how much ethanol that can be broken down. Active ADH enzyme and $NAD^+$ are encapsulated in liposomes, and they are shown to be active. Liposomes were prepared with the following procedure: 40 mg 1-palmitoyl-2-oleoyl-sn-glycero-3-phosphocholin (POPC) was dissolved in 1 mL chloroform and split in two 4 mL glass vials. A glass pipette was used. Can if needed be stored O/N on -20°C. The two vials are placed in a fume hood and left for 15 minutes under a weak nitrogen gas stream to evaporate the chloroform. After evaporation under nitrogen gas stream, the chloroform is evaporated further for two hours in vacuum at a pressure of 0.8 mbar. This is done instead of using a rotary evaporator. After complete evaporation of the chloroform the lipids are hydrated to form liposomes. This is done by slowly adding 2 mL buffer. For later measurements a negative control sample should also be prepared. Such a sample should lack either the enzyme or NAD+ in the hydration buffer. The samples are frozen in liquid nitrogen and afterwards thawed in water at room temperature (20 °C). If increased the enzyme will be likely to denature. The freeze/thaw cycle is repeated seven times to increase unilamellar liposome formation. To obtain a more uniform liposome sample extrusion were done at 800 nm. This size secures several ADH enzymes and $NAD^+$ molecules inside each liposome. Extrusion was performed with a handheld Mini-Extruder from Avanti Polar Lipids, Inc. The extruder was first washed in ethanol and milli-Q water. Then the liposome sample were pressed through once with an 800 nm pore filter. The liposome size was then determined with dynamic light scattering (DLS) using a Zetasizer Nano ZS Size Analyzer from Malvern Panalytical. As background a 0.22 $\mu$m filtrated a 40 mM Tris-HCL buffer pH 8.5 was used. Then 10 $\mu$L of the sample was diluted in 990 $\mu$L filtrated buffer. The lipid concentration was not determined, but this can be done using an inductively coupled plasmamass-spectrometry (ICP-MS). 50 mM Tris-HCL at pH 8.5 were used as a buffer. The concentrations of ADH and $NAD^+$ were varied. In first preparation round a concentration of 2 mg/mL ADH and 30 mM $NAD^+$ was used. Empty liposomes were prepared from a hydration buffer without ADH and $NAD^+$.

In a second liposome preparation round two glass vials with 20 mg POPC each were hydrated with hydration buffer containing 2 mg/mL ADH and 30 mM $NAD^+$. Another two glass vials were hydrated with 0.5 mg/mL ADH and 100 mM $NAD^+$ hydration buffer, and two additional glass vials were hydrated with 0.5 mg/mL ADH and 0 mM $NAD^+$. Calculations to ensure that we had proper amounts of enzymes and NAD+ molecules in each liposome we calculated theoretically how many units were in each liposome structure. With a concentration of 2 mg/mL ADH and 30 mM NAD+ and a liposome size of 800 nm we expect to have around 2152 ADH enzyme molecules and +4 million molecules $NAD^+$. This is calculated with the following formula:

$$(r^3_{lipo} * 4 * \pi / 3) * [molecule] * 6.022 * 10^{23} \; units \; mole = units \; in \; one \; liposome$$

Here $r_{lipo}$ is the expected radius of the liposome, [molecule] is the concentration of the given molecule in the hydration buffer, and $6.022 * 10^{23}$ is Avogadro's constant. Desalting were carried out with GE Healthcare PD-10 columns. The gravity protocol was followed. 50 mM Tris-HCl buffer at pH 8.5 were used as the equilibration buffer.

The flow-through was collected in 1 mL elutions and absorbance at 280 nm was measured in a NanoDrop 2000c spectrophotometer from Thermo Scientific. We expect both the free enzyme, the free NAD+ and the liposomes containing NAD+ and proteins to absorb light at this wavelength. Enzyme activity NADH measurement in flat bottom 96-well plates where 200-300 μL sample was added. Samples were preferably added in duplicates or triplicates.

Then the absorbance at 340 nm was measured in a Tecan Spark 20M multimode microplate reader and incubated at 25 °C. A measurement was taken every minute for at least 30 minutes. Increasing absorbance, indicates increasing NADH concentration which again indicates enzyme activity. This is due to the cofactor NAD+ being converted to NADH when ADH catalyses ethanol degradation.

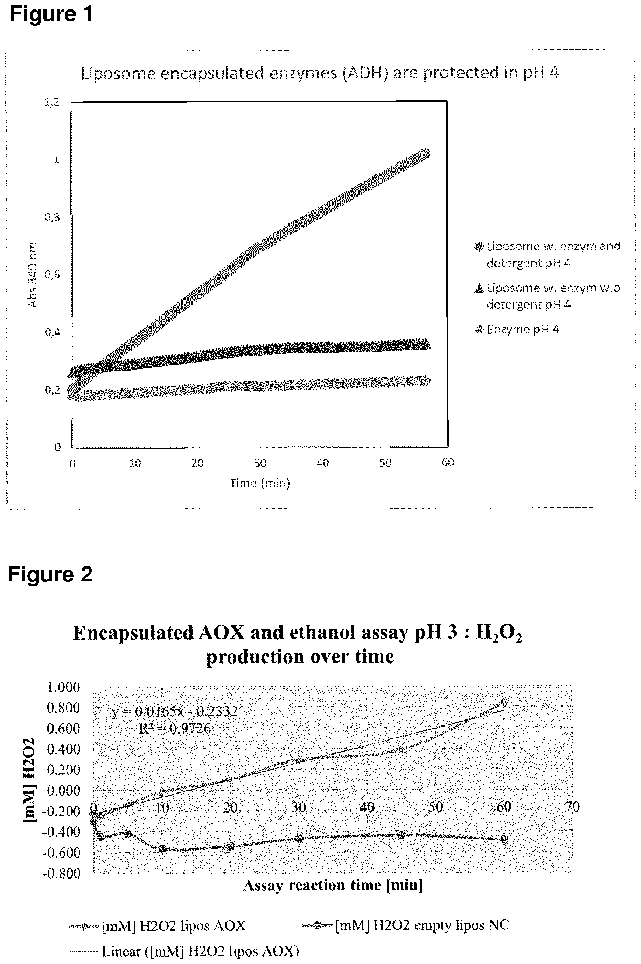

Figure 1 shows results from ethanol degradation by liposomes that enclose ADH and NAD+. The lower curve clearly shows that ADH is stable when encapsulated in a liposome and incubated at pH 4 for 15 minutes.

All samples were incubated at pH 4 for 15 minutes. The absorbance was measured at 340 nm and is proportional to the NADH concentration which is produced as ethanol is degraded by ADH concomitant with the conversion of NAD+ to NADH. In light blue free ADH incubated at pH 4 is shown. Upper and middle curves are from liposomes encapsulating ADH incubated at pH 4 with and without the detergent.

Example 2 - Liposome containing AOX that degrades ethanol.

Figure 2 shows the time-dependent production of $H_2O_2$ from oxidation of 50 mM ethanol catalysed by liposome encapsulated AOX in phosphate buffer, pH 3. (upper line with symbols). Average $H_2O_2$ concentration produced over time with empty liposomes (lower line with symbols). Empty liposomes mean liposomes that does not contain any enzymes. Hence, this can be considered a negative control.

20 mg of POPC were dissolved in 0.5 mL dichloromethane. The phospholipid solution was left to evaporate completely, then additionally left in a vacuum for one hour to ensure total dichloromethane evaporation. The evaporation process formed a white thin film of phospholipids. The thin film was slowly rehydrated with a small amount of hydration buffer (100μL 0.1 M phosphate buffer, pH 7.5) containing 100U/mL pAOX enzymes. Later, after adding the remaining buffer (1.9 mL) the liposome solution was mixed several times, every 15 minutes, by flicking the vial. Afterwards, the liposomes went through a freeze-thaw cycle seven times in liquid nitrogen and 25-30 °C water bath, respectively. After the last freeze step, the samples were stored at -20 °C until the extrusion step. The extruded liposomes were purified by first centrifuging at 7000 crf for six minutes. After discarding the supernatant, the pellet was resuspended in 1 mL wash buffer (50 mM phosphate buffer, pH 7.5) and centrifuged again at 7000 crf for six minutes. The washing step was repeated three times. Lastly, the pellet was resuspended in the 500 μL 0.1 M phosphate buffer, pH 3. Then ethanol degrading activity was tested.

Example 3 - ADH and ALDH activity outside liposome (free)

Figure 3 shows the absorbance measured at 340 nm over time. The absorbance corresponds to NADH production. Samples were measured in triplicates each minute for 5 minutes. The different enzyme samples were dissolved in Tris-HCl buffer at pH 8.5 containing NAD+ and ethanol. The linear trendline is shown for each sample. This clearly shows that ALDH can break down acetaldehyde to acetate.

To determine the synergy between ADH and ALHD initial velocities were measured in the following way. A sample of ADH, ALDH, and ADH+ALDH was prepared. Each well contained 0,1 U ADH and/or 0,5 U ALDH. All wells contained final concentrations of 0,1% (17.2 mM) ethanol, 12 mM NAD+, and 50 mM Tris pH 8.5 buffer. Absorbance was measured at 340 nm in 3 minutes with an interval of 1 minute. A sample of ADH, ALDH, and ADH+ALDH was prepared. Each well contained 0,02 U ADH and/or 0,1 U ALDH. All wells contained final concentrations of 0,01% (1.72 mM) ethanol, 6 mM NAD+, and 50 mM Tris pH 8.5 buffer.

The experiment was started simultaneously by the addition of ethanol with a multichannel pipette. The experiment was started simultaneously by the addition of ethanol with a multichannel pipette. Absorbance was measured at 340 nm with intervals of 2-10 minutes for 800 minutes. Both experiments were performed at room temperature (RT) and biologically duplicated and performed with technical triplicates.

Example 4 - Stability of enzymes in liposomes at acidic pH

Referring to the results in Figure 4, the x-axis shows how many minutes the enzymes were treated at pH 2 in 50 mM phosphate buffer. The y-axis shows the retained activity in % of 0 minutes at pH 2. After 30 minutes at pH 2, V2 retains ~100% activity vs native enzymes 0,2%. V2 liposome consists of 30% cholesterol and 70 % POPC, while V1 consists of only POPC in the membrane. In all cases the enzyme is ADH. The native enzyme (black, left bar) is not encapsulated and close to 0% activity remains after just half a minute at pH 2. V1 liposomes (middle bar) and V2 liposomes (right bar) with ADH shows activity after 60 minutes at pH 2. Stability is tested by the standard activity assay according to the encapsulated enzyme. First the sample is removed from incubation in pH 2 by centrifugation of the sample followed removal of pH 2 buffer with the pipette. Then washing with a 50 mM pH 7.5 phosphate buffer is performed twice and the liposome sample is finally resuspended in 50 mM pH 7.5 phosphate buffer. In this case liposomes are then dissolved by addition of sodium cholate, and then the remaining activity of the released enzymes can be tested. The activity test for ADH is absorbance measurement at 340 nm in a solution containing $NAD^+$ and ethanol.

Example 5 - Activity of peroxidase

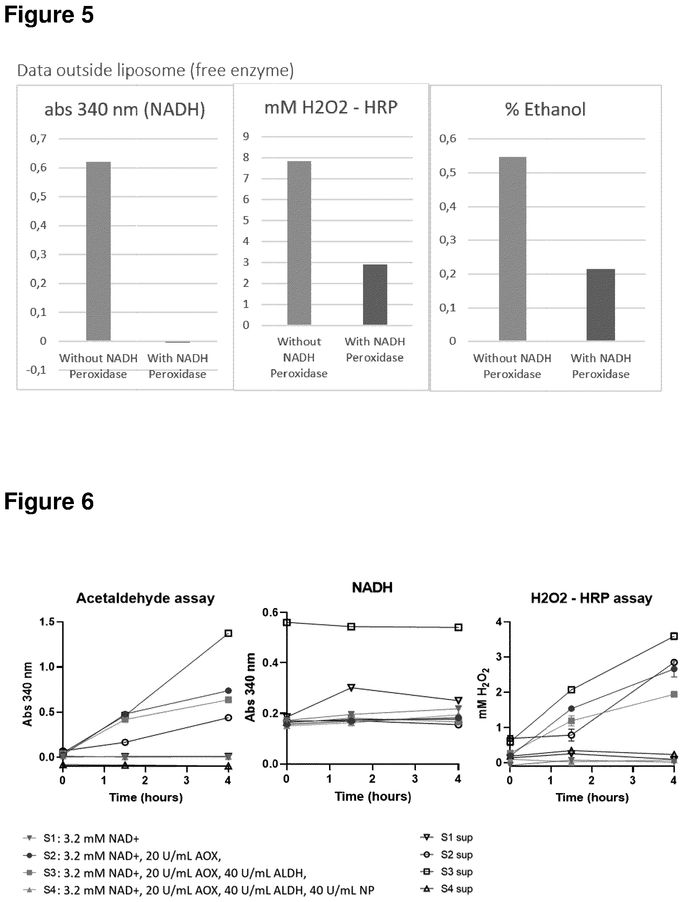

Figure 5 shows results from experiments with ALDH, AOX and NADH peroxide. Both samples contained ALDH, AOX, 1% ethanol, 40 mM phosphate buffer pH 7.5, and limiting amount of $NAD^+$. One sample contained NADH peroxidase (right bar), and the other did not (left bar). After 4 hours NADH (absorbance 340 nm), $H_2O_2$ (mM $H_2O_2$ produced), and ethanol (in %) were measured. NADH was measured directly with absorbance, $H_2O_2$ and ethanol with different coupled enzyme assays.

As expected, it is shown, that NADH is produced only in the system without NADH peroxidase and there is less hydrogen peroxide produced as well. Additionally, more ethanol is degraded with the addition of NADH peroxidase. This must be due to the fact that the addition of NADH peroxidase regenerates $NAD^+$ which enables ALDH to degrade acetaldehyde (produced by AOX) to acetate. This changes the equilibrium and reaction rates of the reaction driving ethanol degradation.

Example 6 - NADH peroxidase can be used to regenerate $NAD^+$ inside liposomes.

Figure 6 shows liposomes with an enzyme system that were tested at pH 3. The enzyme system is composed of AOX, ALDH, and NADH peroxidase (NP). The supernatant that consists of the hydration buffer was tested at pH 7.5. In both the ALDH and NADH assay absorbance was measured at 340 nm and values are shown on the y-axis. For the HRP assays measuring peroxide the concentration of peroxide in mM is shown on the y-axis. The x-axis shows the time in hours. For NADH and acetaldehyde assay technical singlets was measured. For the HRP assay technical duplicates was measured.

In this experiment, a NADH peroxidase from *Streptococcus faecalis* expressed in *E. coli* was tested. The enzyme is relevant as it converts NADH and peroxide to $NAD^+$. By using this enzyme together with ALDH we ensure a continuous supply of $NAD^+$. See Figure 6 for an illustration of this. The results of the four samples are shown in this figure. Here four different samples were prepared: The samples were measured at 0, 1.5 and 4 hours absorbance measurements at 340 nm to measure NADH production, the HRP assay that measures peroxide, and an acetaldehyde assay. For the acetaldehyde assay ALDH and $NAD^+$ were added to the sample. If any acetaldehyde is present, it should produce NADH which will absorb light at 340 nm. Acetaldehyde, NADH, and peroxide concentrations were as expected in the different samples. Acetaldehyde should only be produced in samples 2 and 3 for both the supernatant and the liposomes. NADH should only be produced in the supernatant of sample 3, not the liposomes as it will be trapped inside the liposomes.

Peroxide should be produced mainly in sample 2 and sample 3 which indeed is the case. This experiment should be repeated with the incorporation of the acetate kit from Abcam. This will prove that acetate is produced mainly in sample 4 and show that the NADH peroxidase does regenerate $NAD^+$ which allows for further ALDH catalysis of acetaldehyde to acetate.

Example 7 - Ethanol degradation (acetate formation) by the non-encapsulated enzyme system.

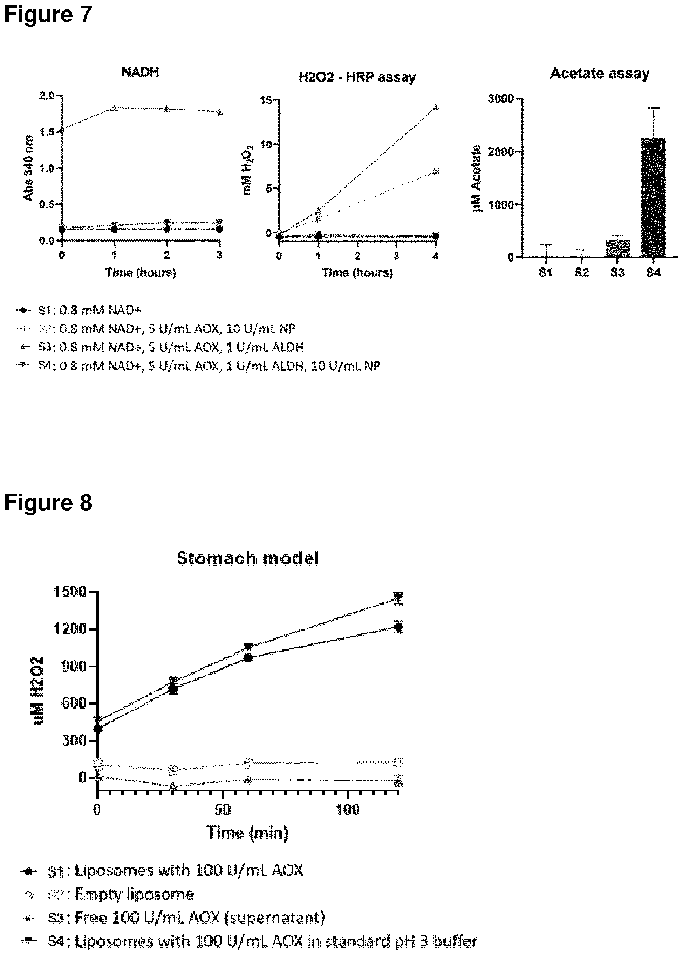

In Figure 7, an enzyme system, composed of AOX, ALDH and NADH peroxidase (NP), in pH 7.5 non-encapsulated is tested for its ethanol degradation. To the left in Fig 7 is shown the absorbance measurement at 340 nm. This should measure the formation of NADH. NADH measurements were performed in singlets. In the middle of Fig 7 results from peroxide measurement with the HRP assay is shown. To the right of Fig 7 is shown the acetate kit measurements which were taken after 4 hours of sample incubation in ethanol. Peroxide and acetate concentrations were measured with technical duplicates. In the experiment, different samples were tested for their ability to produce acetate. The results are shown in the figure below. As expected NADH was only produced in sample 3. This is due to the presence of both ALDH and $NAD^+$, without NADH peroxidase being present. NADH at time 0 is quite high in S1. This is due to the time it takes before actually starting the measurement, where the enzymes already will have produced some NADH. HRP was detected in sample 2 and 3 as these contain AOX without the NADH peroxidase being present. In sample 4 no NADH or peroxide were measured which is believed to be due to the addition of the NADH peroxidase. A one-way ANOVA was performed and the P-value testing whether the samples are equal was <0.0001. Multiple comparisons between the different means were performed. The p-value for the difference in acetate production between samples 3 and 4 was 0.013. Hence, the amount of acetate in sample 4 was significantly higher than in sample 3. This is most likely due to the regeneration of $NAD^+$ by NADH peroxidase. The difference was even more significant between sample 4 compared to samples 1 and 2. In sample 3, $324 \pm 99.3 \, \mu M$ acetate was produced and in sample 4, $2260 \pm 572 \, \mu M$ acetate was produced.

Example 8 - Testing in a stomach model

A simulated stomach fluid was prepared to better simulate the environment of a human stomach. The increased complexity is important to understand whether any of the stomach content will affect the activity of the encapsulated enzymes. We decided to use FaSSGF from biorelevant. This is a preferred choice by several pharma companies and others, simulating the fasted state gastric fluid.

In Figure 8, $\mu$M peroxide is shown on the y-axis and on the x-axis the time in minutes. Measurements were done with biological and technical duplicates. Sample 1 and 4, respectively produced $1220\pm49.74$ $\mu$M $H_2O_2$ and $1453\pm46.50$ $\mu$M $H_2O_2$.

Stomach enzymes are not included in the FaSSGF. To better simulate the stomach fluid pepsin was added. Like stomach models used in other studies, we added 2600 U/mL pepsin in the following experiment. Four different samples were prepared and added to the simulated stomach model containing ethanol. The stomach model was prepared by 100 $\mu$L of the different samples with 200 $\mu$L stomach fluid and 700 $\mu$L 1 % ethanol in citric acid buffer pH 3.

In the stomach model, the AOX liposomes in simulated stomach fluid showed almost the same activity as the control in the pH 3 citric acid 80 mM buffer. In sample 1, $1220\pm49.74$ $\mu$M $H_2O_2$ was produced and in sample 4, $1453\pm46.50$ $\mu$M $H_2O_2$ was produced. No activity was measured for both stomach models in sample 2 and 3. This was as expected as these were negative controls. This tells us that the liposome system performs well in terms of protection of enzymes from salts, acid, proteases.

Example 9 - Synergy between AOX and catalase in ethanol degradation.

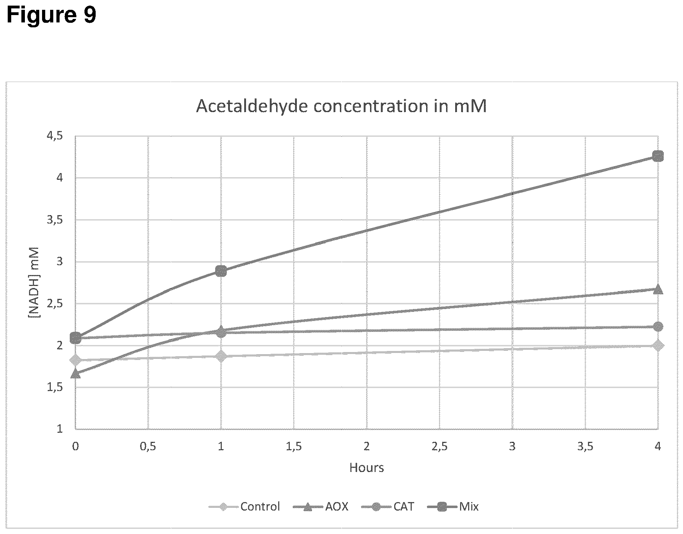

Figure 9 shows the reaction rate in terms of ethanol degradation by liposomes loaded with 100 U/mL AOX, 400 U/mL catalase (CAT), and the combination of AOX and CAT (MIX). The error bars illustrate the standard deviation. NADH mM produced pr hour. Acetaldehyde concentration in molar on the y-axis.

During the conversion of ethanol by AOX, hydrogen peroxide was formed. Its accumulation inactivated AOX, hence, removal of it was needed. Adding catalase into the liposome removed the produced hydrogen peroxide, which prevented the inactivation of AOX. This resulted in a higher ethanol degradation as AOX was able to keep catalysing the oxidation of ethanol. The used measured method quantifies the amount of produced acetaldehyde in a solution by using ALDH and $NAD^+$ and measuring on produced NADH.

Produced acetaldehyde is shown which is equivalent to ethanol degradation. When catalase was present together with AOX it more than doubled the reaction rate and hereby the NADH production.

**References**

A. Akbarzadeh, R. Rezaei-sadabady, S. Davaran, S. W. Joo, and N. Zarghami, "Liposome: classification , preparation , and applications," pp. 1-9, 2013.

Yoshimoto et al.: "Liposomal Encapsulation of Yeast Alcohol Dehydrogenase with Cofactor for Stabilization of the Enzyme Structure and Activity", BIOTECHNOL. PROG., 2008, Vol. 24, p 576-582.

US 2021/0213107

**Claims**

1. A composition comprising a liposome and/or a polymersome, the liposome and/or the polymersome enclose an enzyme capable of degrading ethanol, wherein the liposome and/or the polymersome fulfils at least one of the following criteria:

   i. wherein the concentration of enzyme is above 100 U/ml composition; and/or
   ii. wherein the liposome and/or the polymersome comprises a particle size of at least 400 nm;

2. The composition according to claim 1, wherein the composition is capable of degrading in the range of 50-5000 mM ethanol per hour.

3. The composition according to anyone of claims 1 or 2, wherein the liposome and/or the polymersome comprises a particle size above 400 nm and the liposome and/or the polymersome enclose an enzyme capable of degrading ethanol, and wherein the liposome and/or the polymersome is provided with a concentration of enzyme above 100 U/ml composition.

4. The composition according to anyone of claims 1-3, wherein the liposome and/or polymersome:

- having an inside pH above pH 6.0, such as pH 6.5 or above, e.g. pH 7.0 or above, such as pH 7.5 or above, e.g. pH 8.0 or above, such as pH 8.2 or above, e.g. pH 8.5 or above;
- being stable when subjected to an outside environment having a pH below pH 5.5 or below, e.g. at pH 5.0 or below, such as pH 4.5 or below, e.g. pH 4.0 or below, such as pH 3.5 or below, e.g. pH 3.0 or below, such as below pH 2.5, e.g. below pH 2.0); and
- maintain the stability for a period of 20-300 minutes, such as for a period of 25-240 minutes, e.g. for a period of 30-180 minutes, such as for a period of 35-160 minutes, e.g. for a period of 40-140 minutes, such as for a period of 45-120 minutes, e.g. for a period of 50-100 minutes, such as for a period of 55-90 minutes, e.g. for a period of 58-80 minutes, such as for a period of 60-70 minutes.

5. The composition according to anyone of claims 1-4, wherein the composition is a liquid composition, in particular the liquid composition is an aqueous composition.

6. The composition according to anyone of claims 1-5, wherein the composition is a dried composition, a powdered composition or a pill/tablet comprising the composition.

7. The composition according to anyone of claims 1-6, wherein the liposome and/or polymersome comprises at least one sterol.

8. The composition according to anyone of the present claims, wherein the enzyme capable of degrading ethanol is alcohol oxidase (AOX).

9. The composition according to anyone of the preceding claims, wherein the liposome and/or the polymersome comprises an enzymatic activity in the range of 1-500 U/mg protein.

10. The composition according to anyone of the preceding claims, wherein the liposome comprises phosphatidylcholine obtained from soy, sunflower and/or eggs.

11. The composition according to anyone of the preceding claims, wherein the liposome and/or the polymersome is provided with one or more surface modifications.

12. The composition according to anyone of the preceding claims, wherein the one or more surface modifications is PEGylation, coating (e.g. using polysaccharides), conjugation or absorption of proteins and/or peptides, lipid bilayer modifications, or a combination hereof.

13. The composition according to anyone of the preceding claims, wherein the liposome and/or the polymersome is configured to mainly execute the degradation of ethanol in the stomach of a human.

14. A unit dose comprising a composition according to anyone of claims 1-13, wherein the unit dose comprising an amount of enzyme, enclosed in a liposome and/or a polymersome, of 50 U/unit dose or above.

15. Use of a composition according to anyone of claims 1-13, for the degradation of ingested ethanol in the stomach.

# Figure 1

Liposome encapsulated enzymes (ADH) are protected in pH 4

# Figure 2

**Encapsulated AOX and ethanol assay pH 3 : $H_2O_2$ production over time**

$y = 0.0165x - 0.2332$
$R^2 = 0.9726$

[mM] H2O2 lipos AOX     [mM] H2O2 empty lipos NC

Linear ([mM] H2O2 lipos AOX)

## Figure 3

ALDH interacts synergistically with ADH

## Figure 4

# Figure 5

Data outside liposome (free enzyme)

| abs 340 nm (NADH) | mM H2O2 - HRP | % Ethanol |

# Figure 6

Acetaldehyde assay — NADH — H2O2 - HRP assay

-▼- S1: 3.2 mM NAD+
-●- S2: 3.2 mM NAD+, 20 U/mL AOX,
-■- S3: 3.2 mM NAD+, 20 U/mL AOX, 40 U/mL ALDH,
-▲- S4: 3.2 mM NAD+, 20 U/mL AOX, 40 U/mL ALDH, 40 U/mL NP

-▽- S1 sup
-○- S2 sup
-☐- S3 sup
-△- S4 sup

# Figure 7

**NADH**

**H2O2 - HRP assay**

**Acetate assay**

S1: 0.8 mM NAD+
S2: 0.8 mM NAD+, 5 U/mL AOX, 10 U/mL NP
S3: 0.8 mM NAD+, 5 U/mL AOX, 1 U/mL ALDH
S4: 0.8 mM NAD+, 5 U/mL AOX, 1 U/mL ALDH, 10 U/mL NP

# Figure 8

**Stomach model**

S1: Liposomes with 100 U/mL AOX
S2: Empty liposome
S3: Free 100 U/mL AOX (supernatant)
S4: Liposomes with 100 U/mL AOX in standard pH 3 buffer

# Figure 9

Europäisches
Patentamt

European
Patent Office

Office européen
des brevets

# EUROPEAN SEARCH REPORT

**Application Number**

EP 24 16 2428

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2021/213107 A1 (MOSER FELIX [US] ET AL) 15 July 2021 (2021-07-15) * figure 1 * * example 4 and 5, page 12 * | 1-15 | INV. A61K9/127 A61K47/69 C12N9/04 |
| X | WO 2013/006763 A1 (UNIV CALIFORNIA [US]; YANG OTTO O [US] ET AL.) 10 January 2013 (2013-01-10) * paragraph 31, page 11-12 * * Figures 13A-13C * | 1-15 | |
| X | CN 114 848 841 A (UNIV SHANDONG; SUZHOU RESEARCH INSTITUTE SHANDONG UNIV) 5 August 2022 (2022-08-05) * figure 1 * * paragraph 6, page 3 * | 1-15 | |
| X | CN 116 218 831 A (BEIJING YIXING BIOLOGICAL TECH CO LTD; UNIV NANKAI ET AL.) 6 June 2023 (2023-06-06) * claim 1-34 * | 1-15 | TECHNICAL FIELDS SEARCHED (IPC) |
| X | US 2020/405823 A1 (LU YUNFENG [US] ET AL) 31 December 2020 (2020-12-31) * figure 1a-b * * paragraph 6, page 1 * * page 5-6 * | 1-15 | A61K C12N |

-/--

The present search report has been drawn up for all claims

| Place of search | Date of completion of the search | Examiner |
|---|---|---|
| The Hague | 22 August 2024 | Leitner, Nikolaus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HE HAISHENG ET AL: "Adapting liposomes for oral drug delivery", ACTA PHARMACEUTICA SINICA B, vol. 9, no. 1, 1 January 2019 (2019-01-01), pages 36-48, XP093195237, ISSN: 2211-3835, DOI: 10.1016/j.apsb.2018.06.005 Retrieved from the Internet: URL:https://batavia.internal.epo.org/citen pl/citation/prod/pdf/214a6356-1eb3-3352-b1 6e-93dbf115337a.pdf> * the whole document * | 1-15 | |
| A | ROWLAND R N ET AL: "The stability of liposomes in vitro to pH, bile salts and pancreatic lipase", BIOCHIMICA ET BIOPHYSICA ACTA - LIPIDS AND LIPID METABOLISM, ELSEVIER SCIENCE BV. AMSTERDAM, NL, vol. 620, no. 3, 5 December 1980 (1980-12-05), pages 400-409, XP023372356, ISSN: 0005-2760, DOI: 10.1016/0005-2760(80)90131-9 [retrieved on 1980-12-05] * the whole document * | 1-15 | TECHNICAL FIELDS SEARCHED (IPC) |

The present search report has been drawn up for all claims

| Place of search | Date of completion of the search | Examiner |
|---|---|---|
| The Hague | 22 August 2024 | Leitner, Nikolaus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 2428

This annex lists the patent family members relating to the patent documents cited in the above-mentioned European search report.
The members are as contained in the European Patent Office EDP file on
The European Patent Office is in no way liable for these particulars which are merely given for the purpose of information.

22-08-2024

| Patent document cited in search report | Publication date | Patent family member(s) | | Publication date |
|---|---|---|---|---|
| US 2021213107 A1 | 15-07-2021 | CA | 3162786 A1 | 15-07-2021 |
| | | CN | 114845728 A | 02-08-2022 |
| | | EP | 4087598 A1 | 16-11-2022 |
| | | JP | 2023510302 A | 13-03-2023 |
| | | KR | 20220124710 A | 14-09-2022 |
| | | US | 2021213107 A1 | 15-07-2021 |
| | | US | 2023364202 A1 | 16-11-2023 |
| | | WO | 2021142192 A1 | 15-07-2021 |
| WO 2013006763 A1 | 10-01-2013 | AU | 2012278832 A1 | 20-02-2014 |
| | | AU | 2012278833 A1 | 20-02-2014 |
| | | CA | 2841075 A1 | 10-01-2013 |
| | | CA | 2841079 A1 | 10-01-2013 |
| | | CN | 103781475 A | 07-05-2014 |
| | | CN | 103781905 A | 07-05-2014 |
| | | EP | 2729135 A1 | 14-05-2014 |
| | | EP | 2729569 A2 | 14-05-2014 |
| | | JP | 2014522649 A | 08-09-2014 |
| | | JP | 2014527514 A | 16-10-2014 |
| | | US | 2014134700 A1 | 15-05-2014 |
| | | US | 2014186436 A1 | 03-07-2014 |
| | | US | 2022133859 A1 | 05-05-2022 |
| | | WO | 2013006762 A2 | 10-01-2013 |
| | | WO | 2013006763 A1 | 10-01-2013 |
| CN 114848841 A | 05-08-2022 | NONE | | |
| CN 116218831 A | 06-06-2023 | NONE | | |
| US 2020405823 A1 | 31-12-2020 | US | 2020405823 A1 | 31-12-2020 |
| | | WO | 2019191672 A1 | 03-10-2019 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210213107 A

**Non-patent literature cited in the description**

- **A. AKBARZADEH** ; **R. REZAEI-SADABADY** ; **S. DAVARAN** ; **S. W. JOO** ; **N. ZARGHAMI**. *Liposome: classification , preparation , and applications*, 2013, 1-9

- **YOSHIMOTO et al.** Liposomal Encapsulation of Yeast Alcohol Dehydrogenase with Cofactor for Stabilization of the Enzyme Structure and Activity. *BIOTECHNOL. PROG.*, 2008, vol. 24, 576-582